# DEMANDE DE BREVET EUROPEEN

(11) **EP 4 632 643 A1**
(43) Date de publication de la demande: **15.10.2025**
(21) Numéro de dépôt: 25170135.5
(22) Date de dépôt: 11.04.2025
(51) Int. Cl.: G06Q 10/0631, G06Q 10/0639, G06Q 50/40

(54) **PROCÉDÉ D'ÉVALUATION DU NIVEAU DE FATIGUE D'UN OPÉRATEUR ET SYSTÈME D'ÉVALUATION ASSOCIÉ**

(30) Priorité: 12.04.2024 FR 2403800
(71) Demandeur: THALES, 92190 Meudon (FR)
(72) Inventeur: DURIEUX, Florence, 33700 Merignac (FR); GRANIER, Lionel, 33700 Merignac (FR); IBANEZ, Vincent, 33700 Merignac (FR)
(74) Mandataire: Lavoix

(57) **Abrégé**

La présente invention concerne un procédé d'évaluation du niveau de fatigue d'un opérateur lors d'une mission, le procédé comprenant les phases suivantes mises en œuvre par un ou plusieurs dispositifs transportables d'évaluation:
- une phase initiale de collecte (P1) mise en œuvre avant la mission et comprenant une étape d'acquisition (110) de données personnelles et physiologiques de l'opérateur ;
- une phase finale de collecte (P3) mise en œuvre après la mission et comprenant une étape d'acquisition (310) de données liées à la mission ;
le procédé comprenant en outre :
- une phase d'analyse (P4) comprenant une étape d'analyse (410) de l'ensemble des données collectées et une étape de détermination (420) du niveau de fatigue de l'opérateur avant et/ou après sa mission via cette analyse.

## Description

La présente invention concerne un procédé d'évaluation du niveau de fatigue d'un opérateur.

La présente invention concerne également un système d'évaluation du niveau de fatigue permettant de mettre en œuvre un tel procédé d'évaluation.

L'invention se situe dans le domaine technique de l'évaluation de la fatigue d'un opérateur devant effectuer une mission. L'opérateur peut être un personnel navigant du domaine aéronautique ou de tout autre domaine où la gestion de la fatigue des opérateurs est un enjeu important. Ces domaines regroupent notamment ceux où une permanence opérationnelle de l'opérateur est nécessaire tout au long de sa mission.

L'invention permet plus particulièrement d'optimiser la gestion du risque lié aux opérateurs dans un domaine critique tel que le domaine aéronautique par rapport à leur état de fatigue réel.

Selon l'état de la technique, la fatigue des opérateurs est généralement analysée lors des campagnes temporaires sur la base de questionnaires permettant de capturer la fatigue subjective ou alors sur la base de déclaration de fatigue individuelle.

Ces deux modes de capture de la fatigue permettent uniquement de déduire une fatigue subjective qui peut être biaisée par une pression culturelle ou celle de l'entreprise. En particulier, il a été observé que les opérateurs ont tendance à sous-estimer leur fatigue.

On connait également l'utilisation des modèles biomathématiques pour prédire le niveau de fatigue des opérateurs.

Toutefois, ces modèles utilisent généralement des données moyennes ou alors des données déclaratives de la part des opérateurs.

Les solutions existantes ne sont pas satisfaisantes dans la mesure où elles se basent principalement sur les données déclaratives et/ou moyennes et ne permettent pas donc d'évaluer de manière objective et fiable le niveau de fatigue des opérateurs.

Le but de la présente invention est de proposer une technique d'évaluation du niveau de fatigue des opérateurs permettant de fournir des résultats objectifs et fiables.

À cet effet, l'invention a pour objet un procédé d'évaluation du niveau de fatigue d'un opérateur lors d'une mission.

Le procédé comprend les phases suivantes mises en œuvre par un ou plusieurs dispositifs transportables d'évaluation :
- une phase initiale de collecte mise en œuvre avant la mission et comprenant une étape d'acquisition de données personnelles et physiologiques de l'opérateur ;
- une phase finale de collecte mise en œuvre après la mission et comprenant une étape d'acquisition de données liées à la mission.

Le procédé comprend en outre une phase d'analyse comprenant une étape d'analyse de l'ensemble des données collectées et une étape de détermination du niveau de fatigue de l'opérateur avant et/ou après sa mission via cette analyse.

L'invention permet ainsi d'évaluer le niveau de fatigue d'un opérateur à partir non seulement des données subjectives mais également des données objectives telles que les données physiologiques de l'opérateur.

En outre, l'invention propose de collecter ces différents types de données avant et après la mission, ce qui permet d'estimer l'impact de la mission sur le niveau de fatigue de l'opérateur.

L'invention permet en outre l'analyse de l'évolution de la fatigue induite par les tâches accomplies en offrant un moyen d'évaluation objectif de la fatigue avant la mission et après celle-ci.

Le niveau de fatigue des opérateurs déterminé par l'invention pourra ainsi être utilisé pour identifier les situations propices à l'émergence de la fatigue et pour alors pouvoir les gérer ou éviter. Cela permet de garantir la sécurité de mission effectuée par les opérateurs.

Selon des modes de réalisation particuliers de l'invention, le procédé comprend une ou plusieurs des caractéristiques suivantes, prises isolément ou suivant toutes les combinaisons techniquement possibles :
- une phase intermédiaire de collecte mise en œuvre pendant la mission et comprenant une étape d'acquisition de données liées à l'opérateur et/ou à la mission par un dispositif mobile autre que le dispositif transportable d'évaluation et/ou par un dispositif embarqué ;
- la phase finale de collecte comprend en outre une étape de récupération des données acquises lors de la phase intermédiaire de collecte par transfert de ces données du dispositif mobile ou du dispositif embarqué au dispositif transportable d'évaluation correspondant ;
- la phase initiale de collecte comprend en outre une étape d'acquisition d'au moins un type de données choisi dans le groupe comprenant :
   - évaluation subjective de la fatigue par l'opérateur ;
   - données sur la planification de ses missions passées ;
   - données sur la nature et la difficulté des missions passées ;
   - données sur le sommeil et siestes de l'opérateur ;
   - données sur les activités passées de l'opérateurs ;
   - données sur la mission à effectuer ;
- la phase finale de collecte comprend en outre une étape d'acquisition d'au moins un type de données choisi dans le groupe comprenant :
   - évaluation subjective de la fatigue par l'opérateur ;
   - données caractéristiques de la mission ;
   - évaluation subjective de la difficulté de la mission ;
- la phase intermédiaire de collecte comprend en outre une étape d'acquisition d'au moins un type de données choisi dans le groupe comprenant :
   - évaluation subjective de la fatigue par l'opérateur ;
   - progression de la mission ;
   - faits marquants de la mission. ;
- la phase d'analyse comprend en outre une étape d'affichage d'une recommandation à l'opérateur déterminée en fonction de son niveau de fatigue ;
- la phase initiale de collecte et la phase finale de collecte sont mises en œuvre par des dispositifs transportables d'évaluation différents communiquant entre eux soit directement ou par l'intermédiaire d'un serveur distant ou via un dispositif mobile utilisé pendant une phase de collecte intermédiaire ;
- lorsque la phase d'analyse est mise en œuvre après la phase finale de collecte, l'étape d'analyse comprend une comparaison des données collectées lors de la phase initiale de collecte et lors de la phase finale de collecte ;
- lorsque la phase d'analyse est mise en œuvre au moins une fois à la suite de la phase initiale de collecte et au moins une fois à la suite de la phase finale de collecte.

L'invention a également pour objet un système d'évaluation du niveau de fatigue d'un opérateur, comprenant des moyens configurés pour mettre en œuvre le procédé tel que défini ci-dessus.

L'invention et ses avantages apparaitront à la lecture de la description qui va suivre, donnée uniquement à titre d'exemple non-limitatif et faite en référence aux dessins annexés sur lesquels :
- [Fig.1] la figure 1 est une représentation schématique d'un système d'évaluation du niveau de fatigue d'un opérateur selon l'invention ;
- [Fig.2] la figure 2 est une vue schématique d'un dispositif transportable d'évaluation faisant partie du système d'évaluation de la figure 1 ;
- [Fig.3] la figure 3 est un organigramme d'un procédé d'évaluation selon l'invention, le procédé étant mis en œuvre par le système d'évaluation de la figure 1 ; et
- [Fig.4] [Fig.5] [Fig.6] les figures 4 à 6 sont différentes illustrations de la mise en œuvre du procédé d'évaluation de la figure 3.

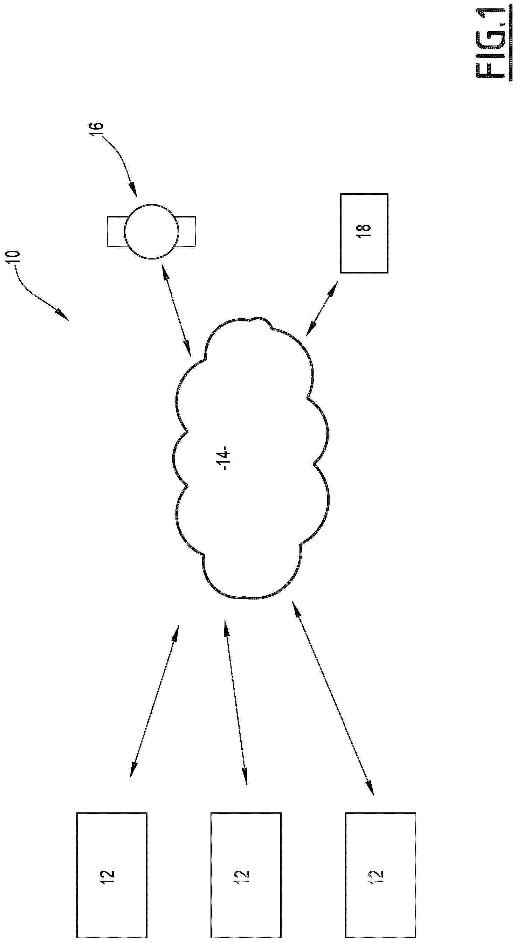

On a en effet représenté sur la figure 1 un système d'évaluation 10 du niveau de fatigue d'un opérateur.

Avantageusement, le système d'évaluation 10 est utilisable dans le domaine aéronautique. Dans un tel cas, l'opérateur fait partie du personnel navigant, notamment du personnel navigant commercial. Selon d'autres exemples, l'opérateur fait partie des opérateurs de planification de vols ou des opérateurs de maintenance ou des opérateurs de contrôle d'aéronef ou des contrôleurs aériens.

Avantageusement, l'opérateur est un pilote apte à piloter un aéronef.

Par aéronef, on entend tout engin volant pilotable à partir du cockpit de celui-ci, comme cela est le cas par exemple d'un avion ou d'un hélicoptère, ou alors à distance de celui-ci, comme cela est le cas par exemple d'un drone.

De manière générale, la notion de l'opérateur peut s'appliquer à toute autre personne effectuant une mission critique, par exemple dans le domaine de transport (ferroviaire ou poids lourd par exemple) ou dans le domaine nucléaire ou spatial, ou dans la médecine.

Comme indiqué précédemment, l'opérateur effectue une mission qui est déterminée par le domaine de son activité.

En particulier, la mission de l'opérateur comprend une pluralité de tâches définies selon les compétences de l'opérateur.

Lorsque l'opérateur est un pilote d'aéronef, sa mission consiste généralement à un pilotage de l'aéronef d'un point de départ à un point de sa destination.

Le système d'évaluation 10 selon l'invention permet de déterminer le niveau de fatigue de l'opérateur.

Pour ce faire, le système d'évaluation 10 comprend au moins un dispositif transportable d'évaluation 12.

Avantageusement, le système d'évaluation comprend plusieurs dispositifs transportables d'évaluation 12 connectés entre eux pour transmettre des données informatiques.

Éventuellement, et comme cela est illustré sur la figure 1, le système d'évaluation 10 comprend un serveur distant 14 permettant l'échange des données entre les différents dispositifs transportables d'évaluation 12. Le serveur distant 14 permet également le stockage des données informatiques issues d'au moins certains dispositifs transportables d'évaluation 12 et dans certains cas, des traitements de ces données.

Dans certains modes de réalisation, le système d'évaluation 10 comprend en outre un dispositif mobile 16 qui a une structure différente de chacun des dispositifs transportables d'évaluation 12, comme cela sera expliqué plus en détail par la suite.

Également dans certains modes de réalisation, le système d'évaluation 10 comprend en outre une pluralité de capteurs 18 installés par exemple dans le poste du travail de l'opérateur tel que le cockpit lorsqu'il s'agit d'un pilote d'aéronef.

Le dispositif mobile 16 et éventuellement les capteurs 18 sont également connectés aux dispositifs transportables d'évaluation 12 directement ou indirectement par exemple via le même serveur 14 comme cela est illustré sur la figure principale.

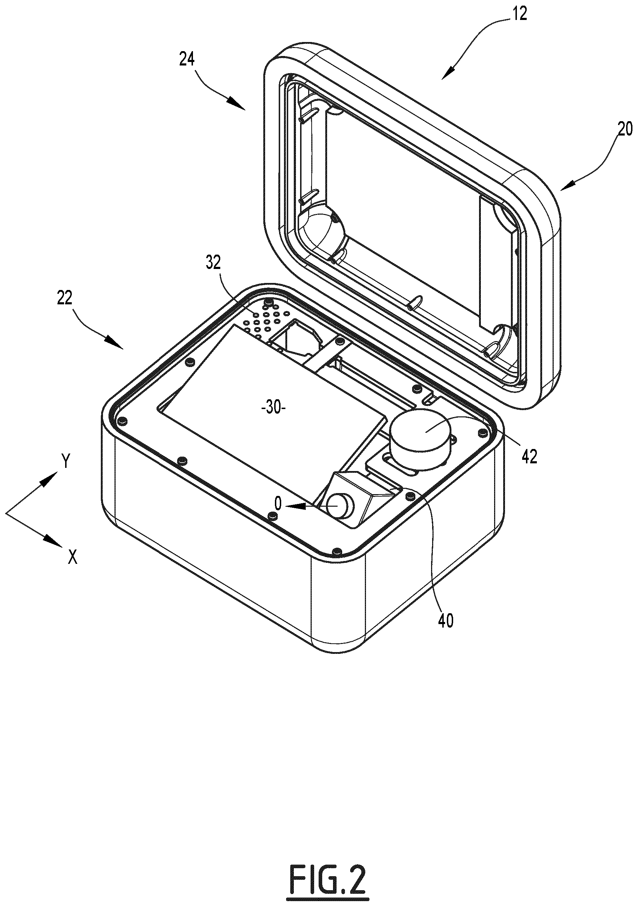

La figure 2 illustre plus en détail un exemple possible de réalisation d'un dispositif transportable d'évaluation 12.

Ainsi, et comme cela est représenté sur cette figure 2, le dispositif transportable d'évaluation 12 présente un boitier 20 intégrant différents composants internes de ce dispositif transportable d'évaluation 12.

En particulier, le boitier 20 se présente par exemple sous la forme d'une valise ou de tout autre objet pouvant être transporté facilement. Dans l'exemple de la figure 2, le boitier 12 est composé de deux demi-coques 22, 24. Le boitier 20 peut comprendre également tout autre dispositif facilitant sa transportation comme par exemple une poignée, des roues, etc.

Au moins l'une des demi-coques, par exemple la demi-coque 24, forme alors une ouverture du boitier 20. Cette demi-coque 24 est mobile entre une position fermée et une position ouverte. Dans la position ouverte, illustrée sur la figure 2, la demi-coque 24 permet alors d'accéder au moins partiellement aux composants internes du dispositif transportable d'évaluation 12.

De manière générale, le boitier 20 comprend une pluralité de composants accessibles par l'opérateur lorsque la demi-coque 24 est dans sa position ouverte et une pluralité de composants inaccessibles par l'opérateur dans toute position de la demi-coque 24.

Parmi les composants accessibles par l'opérateur, le dispositif transportable d'évaluation 12 comprend notamment des moyens d'interaction avec l'opérateur et une pluralité de capteurs.

Les moyens d'interaction avec l'opérateur comprennent notamment des moyens d'interaction visuels tels qu'un écran 30 et des moyens d'interaction auditifs tels que par exemple un haut-parleur 32. L'écran 30 et le haut-parleur 32 sont par exemple intégrés dans une surface intérieure de la demi-coque 22 qui est destinée à être protégée par la demi-coque 24 lorsque celle-ci est dans sa position fermée.

La pluralité de capteurs comprend tout capteur permettant d'acquérir des données physiologiques de l'opérateur.

En particulier, dans l'exemple de la figure 2, la pluralité de capteurs comprend une caméra 40 configurée pour acquérir des images de l'opérateur et un capteur 42 permettant de mesurer le rythme cardiaque de l'opérateur.

La caméra 40 est avantageusement orientée vers l'opérateur ou présente des moyens permettant de l'orienter selon la position de l'opérateur.

Le capteur 42 du rythme cardiaque de l'opérateur est avantageusement amovible du boitier 20 pour par exemple être positionné autour d'un poignet de l'opérateur.

À cet effet, le capteur 42 présente par exemple un bracelet susceptible d'être fixé sur le poignet de l'opérateur et une partie sensible qui est destinée à mesurer le rythme cardiaque de l'opérateur lorsque le bracelet est fixé sur son poignet.

La mesure du rythme cardiaque s'effectue par exemple par la partie sensible par la technique appelée photopléthysmographie, dit PPG. Alternativement, la partie sensible est configurée pour effectuer la mesure du rythme cardiaque à partir d'une analyse de réponse électrique par le poignet de l'opérateur ou par analyse de signaux de radar se propageant dans le poignet de l'opérateur.

Dans certains exemples, le capteur 42 est configuré pour mesurer d'autres paramètres physiologiques de l'opérateur tels que la pression artérielle, l'inspiration de l'oxygène, la sudation, le taux de déshydratation.

Pour la saturation en oxygène, le capteur 42 est par exemple configuré pour émettre en direction de la peau de l'opérateur et recevoir un signal lumineux comprenant au moins deux longueurs d'onde. Une première longueur d'onde correspondant à une longueur d'onde absorbée par les globules rouges saturés, une deuxième longueur d'onde correspondant à une longueur d'onde absorbée par les globules rouges non saturés. Pour déterminer la saturation en oxygène, le capteur 42 est alors configuré pour comparer l'intensité lumineuse reçue en réponse à chacune des deux longueurs d'onde.

De manière générale, le capteur 42 peut se présenter sous la forme d'une montre connectée qui peut alors être stockée dans le boitier 20 lorsque cela est nécessaire ou portée par l'opérateur pour par exemple mesurer son rythme cardiaque.

Bien entendu, les fonctionnalités précitées du capteur 42 peuvent former des capteurs séparés qui peuvent alors être disposés selon toutes configurations possibles à l'intérieur du boitier 20 ou alors sur une surface de celui-ci.

Les composants non accessibles du boitier 20 sont notamment disposés dans la partie intérieure du boitier 20, par exemple dans la partie intérieure de la demi-coque 22 et comprennent notamment un calculateur, une mémoire et un module d'alimentation.

Le calculateur comprend notamment un processeur permettant d'exécuter une pluralité d'applications qui sont stockées par exemple dans la mémoire du boitier.

Le calculateur comprend en outre des moyens de communication avec des dispositifs extérieurs, notamment avec d'autres dispositifs transportables d'évaluation 12 ou alors avec le serveur 14.

Le module d'alimentation permet d'alimenter l'ensemble des composants du dispositif transportable d'évaluation 12. Ce module comprend par exemple une batterie permettant d'alimenter de manière autonome ces composants. Cette batterie peut être associée à un dispositif de recharge permettant de brancher la batterie à un réseau électrique pour la recharger.

Revenant à la description de la figure 1, le dispositif mobile 16 présente tout dispositif permettant d'acquérir et de stocker des données pendant la mission de l'opérateur et de les transmettre à l'un des dispositifs transportables d'évaluation 12.

En variante, le dispositif mobile est seulement propre à stocker les données acquises au cours de la phase initiale de collecte P1. Ce stockage permet avantageusement de transporter ces données d'un point à un autre pendant la mission de l'opérateur. Ces données sont ensuite transmises à l'un des dispositifs transportables d'évaluation 12.

Avantageusement, le dispositif mobile 16 permet également d'acquérir des données physiologiques de l'opérateur. À cet effet, le dispositif mobile 16 comprend par exemple un capteur analogue au capteur 42 décrit précédemment.

Un tel capteur permet par exemple de mesurer le rythme cardiaque de l'opérateur ou d'autres données physiologiques de l'opérateur telles que décrites précédemment.

Le dispositif mobile 16 présente avantageusement une montre connectée.

Dans certains modes de réalisation, le dispositif mobile 16 et le capteur 42 tel que décrit précédemment, présentent la même entité. Dans un tel cas, le dispositif mobile 16 est destiné à être stocké dans le boitier 20 et être porté par l'opérateur lors de sa mission.

Les capteurs 18 présentent le même type de capteurs que ceux décrits en relation avec le dispositif transportable d'évaluation 12. À la différence de ces derniers, ils sont par exemple installés dans le poste de travail de l'opérateur. En outre, chacun des capteurs 18 permet de transmettre les données collectées au moins à l'un des dispositifs transportables d'évaluation 12 via par exemple le dispositif mobile 16 et/ou le serveur distant 14.

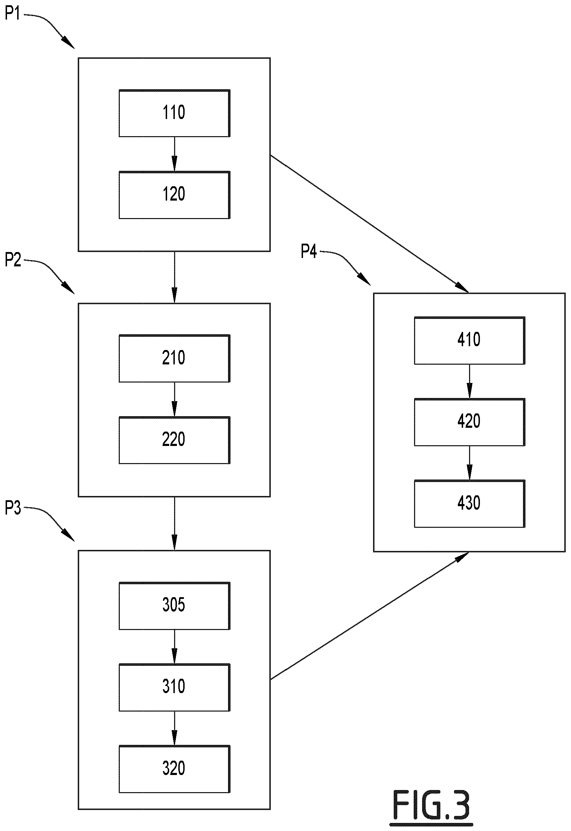

Le système d'évaluation 10 permet de mettre en œuvre un procédé d'évaluation du niveau de fatigue de l'opérateur qui sera désormais décrit en référence à la figure 3 présentant un organigramme de ses étapes.

Le procédé selon l'invention comprend notamment une phase initiale de collecte P1 qui est mise en œuvre avant la mission de l'opérateur.

En particulier, cette phase P1 est mise en œuvre par l'un des dispositifs transportables d'évaluation 12.

Pour mettre en œuvre cette phase P1, le dispositif transportable d'évaluation 12 correspondant est par exemple disposé devant l'opérateur, par exemple lors de la préparation de la mission à venir.

En outre, lorsque le dispositif transportable d'évaluation 12 se présente sous la forme d'une valise, ce dispositif 12 se trouve dans une position ouverte devant l'opérateur de sorte que les capteurs de celui-ci soient orientés vers l'opérateur. L'opérateur peut par ailleurs par exemple mettre sur son poignet le capteur 42 lorsqu'il s'agit d'un capteur amovible.

Le dispositif transportable d'évaluation 12 met ensuite en œuvre une étape d'acquisition 110 de données personnelles et physiologiques de l'opérateur.

En particulier, pour acquérir les données personnelles de l'opérateur, le dispositif 12 interagit avec l'opérateur via l'écran 30 et éventuellement via la caméra 40. Par exemple, le dispositif 12 affiche sur l'écran 30 une série de questions auxquelles l'opérateur doit répondre pour saisir ses données personnelles.

Les données personnelles de l'opérateur comprennent par exemple son identifiant ou son nom et son prénom, une tranche d'âge, etc.

Selon certains exemples, seul l'identifiant de l'opérateur est collecté lors de cette étape. Cet identifiant peut par exemple être anonymisé.

Les données physiologiques de l'opérateur comprennent par exemple l'ensemble des données acquises par la pluralité de capteurs du dispositif 12. Ainsi, par exemple, ces données peuvent par exemple comprendre le rythme cardiaque de l'opérateur acquis par le capteur 40.

Les données physiologiques de l'opérateur peuvent aussi comprendre les images de l'opérateur acquises par la caméra 40 dans un intervalle donné. Cet intervalle peut s'étendre par exemple jusqu'à quelques minutes et peut être mise en œuvre lorsque l'opérateur saisit ses données à l'aide de l'écran 30 ou lorsqu'il prépare sa mission, par exemple en discutant de cette mission avec d'autres opérateurs ou managers.

La phase initiale de collecte P1 comprend en outre une étape d'acquisition 120 de données supplémentaires relatives à l'opérateur et/ou à la mission à venir ou alors aux missions passées.

Par exemple, en ce qui concerne l'opérateur, les données acquises lors de cette étape peuvent comprendre des données sur le sommeil ou sieste effectué par l'opérateur. Ces données peuvent par exemple l'heure du début du sommeil et l'heure de sa fin, la qualité de sommeil, etc.

Les données relatives à l'opérateur peuvent comprendre également des données relatives aux activités effectuées par l'opérateur dans les jours précédents la mission. Ces activités peuvent comprendre des activités en vol et au sol, entrainement, astreinte, maladies, etc.

Enfin, les données relatives à l'opérateur peuvent comprendre aussi une estimation subjective de son niveau de fatigue ressenti ou optionnellement son niveau de stress ou de charge mentale, etc. Pour saisir ces données, le dispositif 12 peut par exemple proposer à l'opérateur d'estimer son niveau de fatigue sur une échelle donnée, son niveau de stress ou de sa charge mentale sur des échelles adaptées à cet effet.

Les informations relatives à la mission à effectuer peuvent par exemple comprendre le poste devant être occupé pendant cette mission (pilote, co-pilote ou autre poste critique par exemple dans le nucléaire ou contrôle aérien), composition de l'équipe (par exemple le nombre de pilotes nécessaire pour le vol à effectuer), la position du jour de travail dans la période (c'est-à-dire le numéro de jour dans la séquence de travail), les informations sur la planification de la mission (mission planifiée, replanifiée et dans ce dernier cas, antériorité de cette replanification), plage de temps de la mission (matinale, en journée, en soirée, nocturne), type d'avion ou tout autre poste de travail sur lequel la mission sera effectuée.

Les données relatives aux missions passées peuvent par exemple comprendre les mêmes données que pour la mission à effectuer. Ces données peuvent également comprendre les difficultés rencontrées lors de ces missions.

Puis, selon certains modes de réalisation, le dispositif 12 met en œuvre une phase d'analyse P4 permettant d'analyser les données collectées lors de la phase initiale de collecte P1.

Cette phase d'analyse P4 comprend notamment une étape d'analyse 410 de l'ensemble de données collectées lors de la phase initiale de collecte P1 et une étape de détermination 420 du niveau de fatigue de l'opérateur avant la mission suite à l'analyse des données collectées lors de la phase initiale de collecte P1.

Pour ce faire, le dispositif 12 analyse les données déclaratives saisies par l'opérateur mais également les données physiologiques acquises par les capteurs du dispositif 12 comme cela a été expliqué précédemment.

Par exemple, l'analyse des images acquises par la caméra 40 peut comprendre la détection du niveau de fatigue de l'opérateur en fonction d'une fréquence de clignotement de ses yeux et/ou en fonction de la fréquence de son bâillement ou d'autres gestes faciaux.

Les données physiologiques peuvent ensuite être superposées avec les données déclaratives pour déterminer avec précision et de manière objective le niveau de fatigue de l'opérateur.

Ce niveau de fatigue peut alors représenter une valeur choisie sur une échelle, par exemple sur une échelle numérique.

Le niveau de fatigue déterminé lors de cette étape 420 peut être affiché à l'opérateur via l'écran 30 ou peut être transmis à son supérieur ou alors peut être stocké sur un serveur externe (par exemple sur le serveur distant 14) de manière sécurisée.

Lorsque l'affichage du niveau de fatigue est effectué sur l'écran 30, cet affichage peut prendre diverses formes choisies en fonction de ce niveau.

Dans certains modes de réalisation, la phase d'analyse P4 peut également comprendre une étape 430 de recommandation qui est mise en œuvre en fonction du niveau de fatigue déterminé lors de l'étape 420.

Par exemple, lorsque le niveau de fatigue est jugé trop élevé, la recommandation donnée par le dispositif 12 peut consister à reporter la mission ou alors à prendre des dispositions nécessaires lors de la mission pour l'effectuer en toute sécurité.

Dans certains modes de réalisation, la phase d'analyse P4 peut également comprendre une étape de transmission de l'ensemble de données collectées au serveur distant 14.

Dans certains modes de réalisation, la phase d'analyse P4 n'est pas mise en œuvre immédiatement après la phase initiale de collecte P1 mais uniquement après la phase finale de collecte P3, comme cela sera expliqué par la suite. Dans un tel cas, les données collectées lors de cette phase initiale peuvent être transmises au serveur distant 14 ou au dispositif mobile 16.

La phase suivante, appelée phase intermédiaire de collecte P2 est mise en œuvre de manière optionnelle après la phase initiale de collecte P1. En particulier, cette phase intermédiaire de collecte P2 est mise en œuvre lors de la mission de l'opérateur par le dispositif mobile 16 tel qu'il a été expliqué précédemment.

En variante ou en complément, cette phase intermédiaire de collecte P2 est mise en œuvre en outre par les capteurs 18, tels qu'expliqués précédemment.

En particulier, cette phase intermédiaire de collecte P2 peut comprendre une étape d'acquisition 210 de données liées à l'opérateur et/ou à la mission par le dispositif mobile 16.

Les données liées à l'opérateur peuvent par exemple comprendre des mesures physiologiques de l'opérateur telles que son rythme cardiaque par exemple tout au long de cette mission.

Les données liées à la mission peuvent par exemple comprendre des informations sur le déroulement de la mission telles que son début, sa durée, sa fin, etc.

La phase intermédiaire de collecte P2 peut également comprendre une étape 220 d'acquisition de données supplémentaires.

Ces données supplémentaires peuvent être relatives à la progression de la mission (décollage, fin de la montée, début de descente, atterrissage) lorsque l'opérateur est un pilote d'aéronef, ainsi que des faits marquants de la mission (panne, turbulence, météo, perturbation de l'équipage).

Les données supplémentaires peuvent également comprendre des estimations subjectives de l'état de fatigue ressenti et optionnellement le niveau de stress ou de charge mentale de l'opérateur.

L'opérateur peut par exemple saisir ces estimations plusieurs fois durant sa mission. Ces estimations peuvent être horodatées.

Le procédé selon l'invention comprend en outre une phase finale de collecte P3 qui est mise en œuvre après la mission. Cette phase finale de collecte P3 peut alors être mise en œuvre suite à la phase intermédiaire de collecte P2 lorsque celle-ci a été mise en œuvre lors de la mission ou alors directement après la phase initiale de collecte P1.

Cette phase finale de collecte P3 est mise en œuvre, tout comme la phase initiale de collecte P1, par un dispositif transportable d'évaluation 12. Ce dispositif peut par exemple être le même que le dispositif 12 utilisé pour mettre en œuvre la phase initiale de collecte P1 ou alors un autre dispositif analogue.

La phase finale de collecte P3 peut comprendre une étape 305 d'acquisition des données acquises lors de la phase intermédiaire de collecte P2 lorsque cette dernière phase a été mise en œuvre pendant la mission de l'opérateur.

Lors de cette étape 305, les données acquises par le dispositif mobile 16 ou alors par les capteurs 18 sont transférées dans le dispositif transportable d'évaluation 12. Cela peut par exemple se faire en connectant ce dispositif 16 directement au dispositif 12. Lorsqu'il s'agit des capteurs 18 disposés par exemple dans le poste de travail de l'opérateur, les données acquises peuvent être transmises en utilisant un serveur externe (par exemple le serveur distant 14).

La phase finale de collecte P3 peut également comprendre une étape d'acquisition 310 de données liées à la mission effectuée par l'opérateur. Ces données liées à la mission peuvent par exemple comprendre le type de mission (entrainement, opérationnel, évaluation, transit), durée de la mission, activités durant la mission (activité de pilotage, gestion de la radio et des autres systèmes), la présence d'un repos pendant la mission et durée de ce repos, les niveaux de perturbation pendant la mission, charge du travail pendant la mission (faible, moyenne, haute).

La phase finale de collecte P3 peut également comprendre une étape d'acquisition 320 de données supplémentaires.

Ces données supplémentaires peuvent par exemple comprendre une évaluation subjective de la fatigue par l'opérateur et une évaluation subjective de la difficulté de la mission.

Ces évaluations peuvent par exemples être saisies par l'opérateur sur une échelle déterminée.

Les données supplémentaire peuvent également comprendre d'autres données caractéristiques de la mission non citées précédemment.

Ces données caractéristiques peuvent peuvent par exemple correspondre à des données techniques relatives au déroulement de la mission.

Ensuite, le dispositif transportable d'évaluation 12 met en œuvre la phase d'analyse P4 telle qu'elle a été expliquée précédemment.

En particulier, à la différences des explications précédentes, l'étape 410 de cette phase comprend l'analyse de l'ensemble des données collectées lors des phases précédentes.

Pour ce faire, le dispositif transportable d'évaluation 12 peut accéder aux données collectées lors de la phase initiale de collecte P1, par exemple sur le serveur distant 14 ou via le dispositif mobile 16. Ainsi, l'étape d'analyse 410 comprend l'analyse de toutes les données collectées lors de toutes les phases de collecte précédentes. Cette étape d'analyse 410 peut comprendre également une comparaison des données collectées lors de la phase initiale de collecte P1 et lors de la phase finale de collecte P3 et éventuellement lors de la phase intermédiaire de collecte P2.

Ensuite, l'étape 420 détermine le niveau de fatigue de l'opérateur en utilisant toutes les données collectées et analysées.

Comme dans le cas précédent, le niveau de fatigue de l'opérateur déterminé à ce stade peut être affiché à l'opérateur via l'écran 30 ou alors transmis à son supérieur.

Également comme dans le cas précédent, la phase d'analyse P4 peut comprendre l'étape de recommandation 430. Dans ce cas, la recommandation donnée à l'opérateur prend en compte le fait que la mission a déjà été effectuée. La recommandation donnée à cette étape peut par exemple consister en quantité d'heures repos conseillée pour l'opérateur.

Comme indiqué précédemment, la phase d'analyse P4 est mise en œuvre soit par le dispositif 12 mettant en œuvre la phase de collecte initiale P1, soit par le dispositif 12 mettant en œuvre la phase finale de collecte P3.

Toutefois, dans un cas général, cette phase P4 peut également être mise en œuvre indépendamment de ces dispositifs, par exemple par un calculateur externe connecté audit dispositif 12 par exemple via le serveur 14 pour collecter les données acquises.

Le serveur 14 peut par ailleurs garder l'ensemble des données collectées et analysées pour une utilisation future de ces données, par exemple pour déterminer une statistique correspondante.

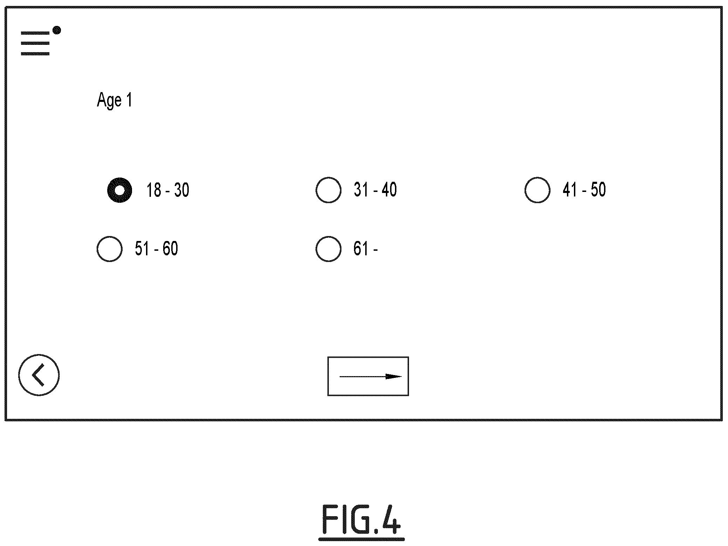

Les figures 4 à 6 illustrent une mise en œuvre de certaines étapes décrites précédemment.

Ainsi, par exemple la figure 4 illustre l'étape 110 d'acquisition des données personnelles de l'opérateur. Lors de cette étape, le dispositif 12 propose notamment à l'opérateur de saisir sa tranche d'âge comme cela est illustré sur la figure 4.

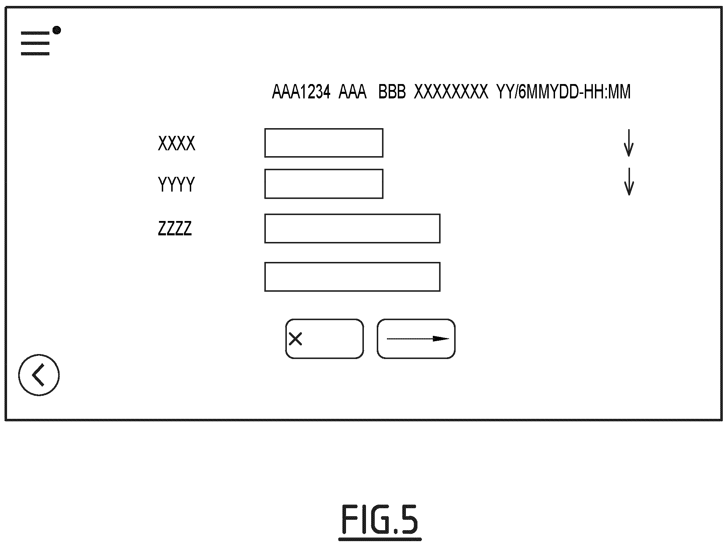

La figure 5 illustre un exemple de la mise en œuvre de l'étape 310 de l'acquisition des données liées à la mission.

Par exemple, lors de cette étape, le dispositif 12 invite l'opérateur à saisir les données météo relatives à la mission. Pour cela, le système 12 propose par exemple des différents choix parmi des options possibles.

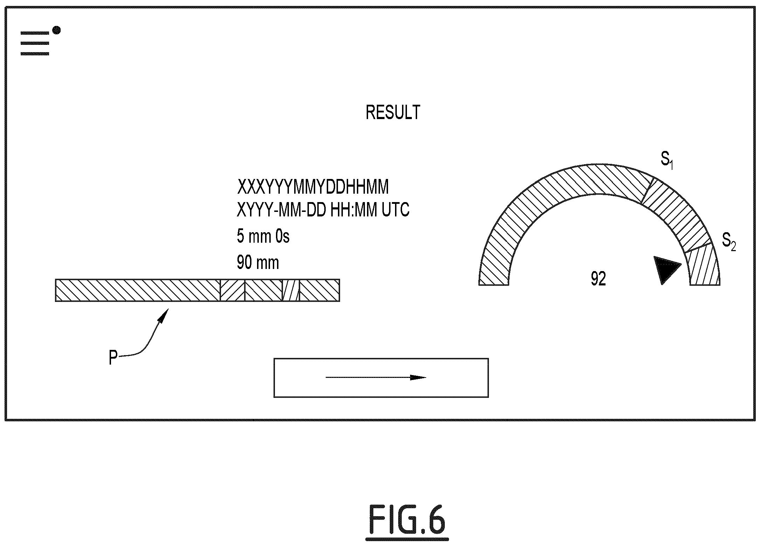

Enfin, la figure 6 illustre une mise en œuvre de l'étape 420 lors de laquelle le niveau de fatigue déterminé par la phase d'analyse P4 est affiché à l'opérateur.

Comme cela est illustré sur cette figure, le niveau de fatigue attribué peut être attribué par exemple sur une échelle de 0 à 100. Cette échelle définit par ailleurs quelques seuils permettant de donner une évaluation rapide du niveau de fatigue. Par exemple sur cette figure, deux seuils S1 et S2 sont déterminés.

Lorsque le niveau de fatigue se retrouve inférieur au seuil S1, le niveau de fatigue est considéré comme normal. Lorsque le niveau de fatigue est compris entre les deux seuils S1 et S2, ce niveau est considéré comme élevé. Lorsque le niveau de fatigue est supérieur au seuil S2, celui-ci est considéré comme très élevé.

La figure 6 illustre également une échelle temporaire P d'évolution du niveau de fatigue, par exemple au cours de la mission.

Bien entendu, de nombreuses autres illustrations de mise en œuvre des étapes précitées sont également possibles.

En variante ou en complément, lorsque l'opérateur est déterminé comme étant fatigué au cours de la phase d'analyse P4, c'est-à-dire que sa fatigue est supérieure au seuil S1, sa fatigue est catégorisée. Sa fatigue appartient alors à un type spécifique déterminé par des causes liées à sa mission. Par exemple, une mission anormalement longue induira une fatigue catégorisée comme étant une fatigue de type long terme ou fatigue chronique. En revanche, un effort intense tel qu'une mission avec de nombreux incidents imprévus induira une fatigue de type charge mentale élevée ou fatigue aigue.

D'autres causes de fatigue sont liées à des évènements extérieurs à sa mission. Par exemple, la fatigue de l'opérateur peut être catégorisée comme étant émotionnelle suite à un évènement personnel. La fatigue de l'opérateur peut également être liée à des conditions médicales ou au manque de sommeil.

La fatigue d'un opérateur peut également être classifiée avec plusieurs types de fatigue lorsque celle-ci remplit plusieurs causes.

Avantageusement, cette variante permet de contextualiser la fatigue d'un opérateur.

## Revendications

1. Procédé d'évaluation du niveau de fatigue d'un opérateur lors d'une mission, le procédé comprenant les phases suivantes mises en œuvre par un ou plusieurs dispositifs transportables d'évaluation (12):
- une phase initiale de collecte (P1) mise en œuvre avant la mission et comprenant une étape d'acquisition (110) de données personnelles et physiologiques de l'opérateur ;
- une phase finale de collecte (P3) mise en œuvre après la mission et comprenant une étape d'acquisition (310) de données liées à la mission ;
le procédé comprenant en outre :
- une phase d'analyse (P4) comprenant une étape d'analyse (410) de l'ensemble des données collectées et une étape de détermination (420) du niveau de fatigue de l'opérateur avant et/ou après sa mission via cette analyse.

2. Procédé selon la revendication 1, comprenant en outre une phase intermédiaire de collecte (P2) mise en œuvre pendant la mission et comprenant une étape d'acquisition (210) de données liées à l'opérateur et/ou à la mission par un dispositif mobile autre (16) que le dispositif transportable d'évaluation (12) et/ou par un dispositif embarqué (18).

3. Procédé selon la revendication 2, dans lequel la phase finale de collecte (P3) comprend en outre une étape de récupération (305) des données acquises lors de la phase intermédiaire de collecte (P2) par transfert de ces données du dispositif mobile (16) ou du dispositif embarqué (18) au dispositif transportable d'évaluation (12) correspondant.

4. Procédé selon l'une quelconque des revendications précédentes, dans lequel la phase initiale de collecte (P1) comprend en outre une étape d'acquisition (120) d'au moins un type de données choisi dans le groupe comprenant :
- évaluation subjective de la fatigue par l'opérateur ;
- données sur la planification de ses missions passées ;
- données sur la nature et la difficulté des missions passées ;
- données sur le sommeil et siestes de l'opérateur ;
- données sur les activités passées de l'opérateurs ;
- données sur la mission à effectuer.

5. Procédé selon l'une quelconque des revendications précédentes, dans lequel la phase finale de collecte (P3) comprend en outre une étape d'acquisition (320) d'au moins un type de données choisi dans le groupe comprenant :
- évaluation subjective de la fatigue par l'opérateur ;
- données caractéristiques de la mission ;
- évaluation subjective de la difficulté de la mission.

6. Procédé selon l'une quelconque des revendications précédentes prise en combinaison avec la revendication 2, dans lequel la phase intermédiaire de collecte (P2) comprend en outre une étape d'acquisition (220) d'au moins un type de données choisi dans le groupe comprenant :
- évaluation subjective de la fatigue par l'opérateur ;
- progression de la mission ;
- faits marquants de la mission.

7. Procédé selon l'une quelconque des revendications précédentes, dans lequel la phase d'analyse (P4) comprend en outre une étape d'affichage (430) d'une recommandation à l'opérateur déterminée en fonction de son niveau de fatigue.

8. Procédé selon l'une quelconque des revendications précédentes, dans lequel la phase initiale de collecte (P1) et la phase finale de collecte (P3) sont mises en œuvre par des dispositifs transportables d'évaluation différents (12) communiquant entre eux soit directement ou par l'intermédiaire d'un serveur distant (14) ou via un dispositif mobile (16) utilisé pendant une phase de collecte intermédiaire (P2).

9. Procédé selon l'une quelconque des revendications précédentes, dans lequel lorsque la phase d'analyse (P4) est mise en œuvre après la phase finale de collecte (P3), l'étape d'analyse (410) comprend une comparaison des données collectées lors de la phase initiale de collecte (P1) et lors de la phase finale de collecte (P3).

10. Procédé selon l'une quelconque des revendications précédentes, dans lequel lorsque la phase d'analyse (P4) est mise en œuvre au moins une fois à la suite de la phase initiale de collecte (P1) et au moins une fois à la suite de la phase finale de collecte (P3).

11. Procédé selon l'une quelconque des revendications précédentes, dans lequel un dispositif mobile (16) autre que le dispositif transportable d'évaluation (12) est propre à stocker, pendant la mission de l'opérateur, les données acquises au cours de la phase initiale de collecte (P1) pour les transmettre à l'un des dispositifs transportables d'évaluation (12).

12. Procédé selon l'une quelconque des revendications précédentes, dans lequel, lorsque l'opérateur est déterminé comme étant fatigué au cours de la phase d'analyse (P4), sa fatigue appartient à un type spécifique déterminé par des causes liées ou non à sa mission.

13. Système d'évaluation (10) du niveau de fatigue d'un opérateur, comprenant des moyens (12, 14, 16, 18) configurés pour mettre en œuvre le procédé selon l'une quelconque des revendications précédentes.
